# EUROPEAN PATENT SPECIFICATION

(11) **EP 3 169 428 B1**
(45) Date of publication and mention of the grant of the patent: **20.03.2019**
(21) Application number: 15747303.4
(22) Date of filing: 17.07.2015
(51) Int. Cl.: B01F 11/00

(54) **MIXING AND DISPENSING APPARATUS, METHOD AND KIT FOR COMBINATION MATERIALS**
MISCH- UND AUSGABEVORRICHTUNG, VERFAHREN UND KIT FÜR KOMBINATIONSMATERIALIEN
APPAREIL, PROCÉDÉ ET KIT DE MÉLANGE ET DE DISTRIBUTION POUR MATÉRIAUX EN COMBINAISON

(30) Priority: 18.07.2014 US 201462026131 P
(43) Date of publication of application: 24.05.2017
(73) Proprietor: Ethicon, Inc, Somerville, NJ 08876 (US)
(72) Inventor: SMITH, Daniel J., Dayton, New Jersey 08810 (US); VAILHE, Christophe, Hillsborough, New Jersey 08844 (US); NORDMEYER, Michael W., Pittstown, New Jersey 08867 (US)
(74) Representative: Carpmaels & Ransford LLP
(86) International application number: PCT/US2015/040843
(87) International publication number: WO 2016/011311

(56) References cited:
- WO-A1-2011/103384
- GB-A- 912 237
- US-A1- 2004 267 272

## Description

### Field

The present invention relates to methods and apparatuses for mixing materials, including combination materials, such as powders, gels, aqueous materials, and other similar materials. The mixing apparatus may also function as a dispensing apparatus.

### Background

Mixing materials to form a resulting combination or mixture is known, and includes various techniques such as hand mixing to electro-mechanical mixing. In certain situations, certain materials cannot be pre mixed due to storage stability issues or when one or more components react to the other or to moisture. For example, in some instances, a powder is to be mixed with a gel or aqueous material, and the powder must be maintained dry to prevent degradation, so pre-mixing is undesired. In other situations, a mixture is to be made but is to be dispensed soon after mixing or after a resting period to obtain a particular viscosity. Still further complications arise due to sterilization methods. For example, some gels may be steam sterilized because they are too easily degraded by radiation, while other materials are sterilized by radiation due to heat or moisture instability.

The mixing of two components, such as a gel and a powder, may be difficult, similar to mixing flour and water. In these instances, the outer layer of the powder wets and the inner layer of the powder remains dry, which leads to clumping and a varied concentration of the mixture. The mixing can be further hindered by the method, as too little speed can allow the mix to set quickly forming a paste, but too much speed can increase pressure pushing out moisture and creating packed powder at the bottom. Incomplete mixing creates clumps and clumps clog the mixer or the applicator/dispenser. In some instances, a dry powder may have an affinity to water, and the gel or other material includes a high concentration of water, leading to potential complications. In some situations, a phenomenon known as gel blocking can interfere with dispensing of mixtures.

GB 912,237 relates to a mixing device that is said to be suitable for mixing materials, such as extrudable mastics, in the container in which they are sold. The mixing device comprises a longitudinally reciprocable driving rod carrying a mixing head associated with means adapted to cause the rod to rotate about its own axis when longitudinally moved in one direction, and which enable it to move longitudinally in the reverse direction without rotation.

US 2004/0267272 relates to a device for mixing and delivering bone cement comprising a mixing cartridge, a mixing device and a delivery gun. The mixing device includes a mixing shaft connected at one end to a mixing blade and at the other end to a power tool to mix liquid and powder components of a bone cement.

WO 2011/103384 relates to various systems for mixing therapeutic agents before and/or during delivery. The systems may comprise a syringe including a mixing means, such as an internal metallic member and an external rotating magnet, such that actuation of the external rotating magnet induces motion of the internal metallic member.

The present invention seeks to resolve various issues involved with mixing components by providing an improved mixing apparatus and method for mixing. The mixing device may further include a dispensing device, thereby allowing mixing and dispensing in one device.

### Summary

The present invention relates to mixing devices and apparatuses, along with components to achieve mixing of multiple components. In one aspect, there is a mixing apparatus for achieving mixing of multiple components, including a mixing chamber having a first end and second end, where the first end is open; and a mixing device secured to the first end, the mixing device including a plunger, a rod, a mixing head having a plurality of angled blades, and a device to control spin of the mixing head, where the mixing head is inserted at least partially into the mixing chamber, where the device to control spin of the mixing head causes the mixing head to spin when the plunger is moved in a first axial direction, and causes the mixing head to avoid spinning when the plunger is moved in a second axial direction.

There is also included methods of mixing, such as a method of mixing two components including having a first component and a second component into a mixing chamber having a first end and second end, where the first end is open; securing a mixing device to the first end, the mixing device including a plunger, a rod, a mixing head having a plurality of angled blades, and a device to control spin of the mixing head, where the mixing head is inserted at least partially into the mixing chamber; and moving the plunger in a first axial direction and in a second axial direction at least one time each, where the device to control spin of the mixing head causes the mixing head to spin when the plunger is moved in a first axial direction, and causes the mixing head to avoid spinning when the plunger is moved in a second axial direction.

There is also included a kit including a mixing chamber having a first end and second end, where the first end is open; a mixing device capable of being secured to the first end, the mixing device including a plunger, a rod, a mixing head having a plurality of angled blades, and a device to control spin of the mixing head, where the mixing head is inserted at least partially into the mixing chamber; a first material to be mixed; and a second material to be mixed.

In other aspects of the invention, which is defined by the features of the independent claims 1, 4 and 7, there is provided a syringe locking system to maintain components together during a mixing process, and is suitable for various size syringes.

### Brief Description of the Figures

Figure 1 depicts an embodiment of a mixing apparatus.
Figure 2 is a close-up view of a mixing head.
Figure 3A is a close-up front view of a mixing head.
Figure 3B is a close-up side view of a mixing head.
Figure 4A is a close-up front view of a mixing head.
Figure 4B is a close-up side view of a mixing head.
Figure 5A is a close-up front view of a mixing head.
Figure 5B is a close-up side view of a mixing head.
Figure 6 is a close-up front view of a mixing head.
Figure 7A is a close-up front view of a mixing head.
Figure 7B is a close-up perspective view of a mixing head.
Figure 8 is a close-up perspective view of a mixing head.
Figure 9A is a close-up perspective view of a mixing head.
Figure 9B is a close-up side view of a mixing head.
Figure 10 is a close-up perspective view of a mixing head.
Figure 11 is a close-up perspective view of a mixing head.
Figure 12 is a close-up perspective view of a mixing head.
Figure 13 is a close-up perspective view of a mixing head.
Figure 14 is a close-up perspective view of a mixing head.
Figure 15 is a perspective view of a mixing apparatus with a safety cover thereon.
Figure 16 is a side perspective view of an assembled mixing apparatus connected to a syringe.
Figures 17 and 18 are views of thread configurations. Figure 17 shows a thread with a 0.64 cm (0.25 inch) pitch. Figure 18 shows a thread with a 2.54 cm (1.0 inch) pitch.
Figures 19A-19F are perspective views of various mixing heads with raised protrusions.
Figure 20 is a depiction of various components useful in a kit.
Figure 21 is a depiction of various components useful in a kit.
Figure 22 is an embodiment showing a compact mixing apparatus.
Figure 23 is a depiction of the compact mixing apparatus of Figure 22 connected to a delivery syringe.
Figure 24 is another depiction of a mixing apparatus and delivery syringe connected to each other.
Figure 25 is a cross-sectional view of the mixing apparatus of Figure 24.
Figure 26 is a side view of the mixing apparatus of Figure 24.
Figure 27 is a front and side view of a mixing head.
Figures 28-34 show various views and depictions of a syringe lock system.

### Detailed Description

The present invention relates to methods and apparatuses for mixing at least two components. The two components may include, for example, powders, gels, aqueous materials, solvents, and combinations thereof. The apparatuses used may include various components, such as a mixing component, a dispensing component, various syringes, and a dispensing tip. Each of these components may be used by themselves, in combination, or as part of a kit. For example, the dispensing component and mixing component may be used by themselves in combination, or the mixing component may be used by itself.

Most materials may be mixed by the present invention by choosing and appropriate mixing blade, and in some cases, the mixing includes a first material in the form of a powder and a second material in the form of a gel. For example, the powder material may include an oxidized regenerated cellulose in the form of particles or fibers, while the gel may include a cellulose, such as carboxymethyl cellulose. Polysaccharides may be useful as the first and/or second materials. Additional components may be mixed in combination, including, for example, solvents and/or liquid carriers, such as saline. The mixer is most useful to easily mix dry material (or dry material that has been wetted with an aqueous material) with very viscous material, such as a gel or cream, which typically is not easily and thoroughly mixed. The use of an aqueous material to wet the powder aids in reducing clumping of the powder, and is therefore preferred but not required in some instances, for example, if the gel has sufficient aqueous material as part of the gel composition.

Attempts to mix a powder and viscous gel together via typical luer lock syringes have commonly failed, are extremely tedious, or are operator dependent, and even successful attempts have only been in small syringe volumes such as 10 ml and smaller. A phenomenon sometimes referred as "gel blocking" was found to prevent an easy and sufficient mixing of these two components by simply expressing one syringe into the other. When "gel blocking" occurs in the luer-lock connector, the force to move material from one syringe to another exceeds that of the human hand and cannot be undone rendering the device useless. As used herein, the term gel blocking refers to a phenomenon that occurs when the swelling of a superabsorbent polymer blocks the passage of fluid into the center of a material, thereby reducing the absorption capacity. The technical challenge solved by the present invention is to mix large and/or small volumes of a first absorbent powder (such as ORC powder) with a gel-type material (such as CMC gel) in a rapid and sufficiently complete manner immediately prior to or at the time of application (such as during a surgical procedure). It is helpful to mix these components directly in a syringe, where the syringe is to be used as the dispensing component.

Shear is a factor to consider for mixing of materials such as powders and gels. Typically shear is the result of the mixing process, resulting in a decrease of the material viscosity, which is sometimes referred to as shear thinning. However, the present mixing system has shown that mixing can be achieved either with limited shear thinning or with shear thinning level depending on the blade configuration and number of strokes used to mix. This allows creation of a thoroughly mixed ORC/CMC gel that has a very high viscosity, and conversely the same mixing apparatus and ORC/CMC mixture by using a different blade configuration may yield a lower viscosity gel. The end result provides for either a high viscosity mixture, which can then be dispensed as a thick gel using an open dispenser, or as a thin creamy gel by restricting a dispenser opening. This is interesting as well as novel and unexpected, since syringe to syringe mixing when possible will typically produce highly sheared gel, which presents as a thin creamy gel. Details of certain ORC/CMC compositions and methods of making and using certain ORC/CMC compositions may be found in U.S. Patent Application No. 62/026,148, filed July 18, 2014, and U.S. Patent Application No. 62/026,156, filed July 18,2014.

The present inventive mixing apparatus and assembly and method can mix a variety of mixture concentrations effortlessly by a user in any size mixing and dispensing syringe. The inventive mixing apparatus is capable of mixing larger quantities of powder (such as ORC powder, which may be wetted with an aqueous material) and gel (such as CMC gel) easier and quicker than previous attempts. This can be achieved in smaller volumes (e.g., 5 or 10 mL) or larger volumes (e.g., 50 mL), but could be scaled up to larger sizes. Further, unlike syringe to syringe mixing, it is easier to mix larger volumes than smaller volumes in the inventive mixer. This may be partly due to the larger blade diameter having higher surface speed, and increased shear. To minimize excessive shear a more open blade structure may be used.

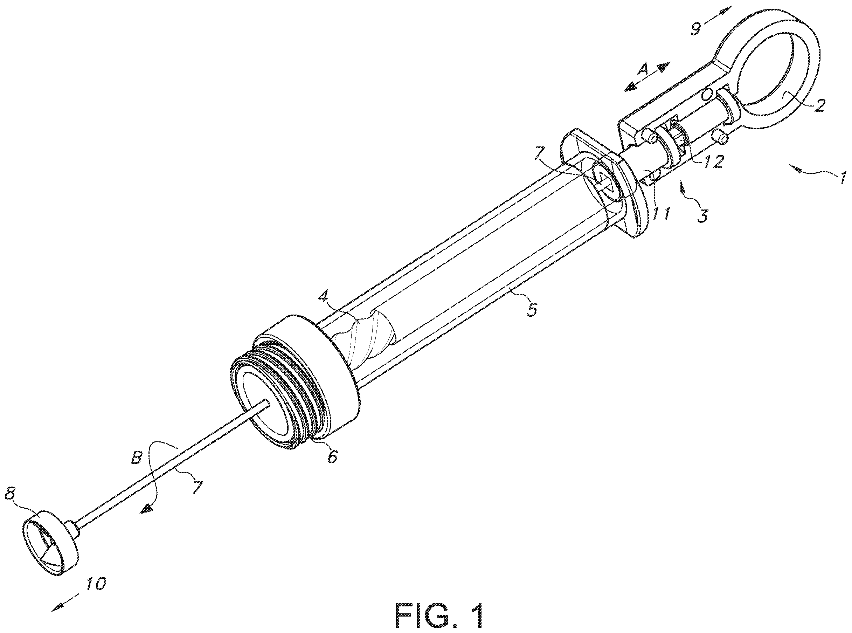

With reference to Figure 1, a mixing component or mixing device is depicted. A mixing device includes a mixing plunger 1, which may include a handle 2. Handle 2 may include, for example, a finger hole, a grip, or other ergonomic feature useful to a user as well as any safety related features. The plunger 1 is capable of moving in an axial direction (A). The plunger 1 may be a push-pull type plunger, or it may move via screwing or turning method. The mixing device may include a detachable or connectable hinge system 3, which will be described in greater detail below. Briefly, the hinge system 3 allows for the mixer to spin a mixing head when moved in a first axial direction, but avoid spinning when moved in the opposite axial direction. This movement is referred to as spin-down/pull-up mixing. Further the hinge system feature, which consists of a hinge housing, hinge and hinge pin, can provide randomization of where the blades starting position will be such that the downward blade path could be different than the previous pathway through the gel. This is particularly useful when less blade fins are used, as well as if a spin-down/spin-up mixing method is used. A mixing device includes a threaded mixing screw 4, which extends axially along the mixing device and is connected to the hinge system 3. The threaded mixing screw 4 may be housed within a mixing housing 5, which is an axially extending, hollow cylindrical body. The threaded mixing screw 4 is preferably releasable connected to the mixing plunger 1, such that when the mixing plunger 1 is pulled axially in the proximal direction (9), the threaded mixing screw 4 is pulled in the proximal direction 9 as well. When the mixing plunger 1 is pushed in the distal direction 10, the threaded mixing screw 4 is rotated in the distal direction.

Connector 11 is securably attached to the proximal end of the mixing screw 4, and includes an opening to receive a hinge pin 12, as will be described below.

Positioned through the end of the threaded mixing screw 4 axially opposite the plunger 1 is a mixing rod 7, which is a rigid cylindrical device. The rod 7 is slidable axially through the mixing screw 7 as well as the mixing body 5. At the end of the rod 7 is a mixing head 8, which will be described in greater detail below. The mixing head 8 may be generally cylindrical, but may have any cross-sectional configuration in which a blade head could spin within the mixing syringe to facilitate mixing, and is secured to the rod 7 such that if the rod 7 rotates, the mixing head 8 rotates simultaneously. If the rod 7 is moved axially, the mixing head 8 moves simultaneously. Plunger 1, threaded screw 4, mixing body 5, and rod 7 are all aligned along the axis A. The plunger 1 and mixing head 8 are connected axially, but not rotatably to each other by the hinge pin 12 and rod 7, where the rod 7 extends from within plunger 1 to the mixing head 8, and through the threaded screw 4. Plunger 1, rod 7 and mixing head 8 are therefore all capable of being moved along axis A, in the proximal direction 9 and the distal direction 10. Threaded screw 4 has an open central axis, through which the rod 7 travels.

At the distal end of the mixing body 5 is an engagement feature 6, which may be a threaded engagement. In some embodiments, the engagement feature 6 may include a male screw-type mechanism, which is designed to mate with a female threaded receiver. In other embodiments, the engagement feature 6 may include a snap-fit mechanism to mate with a companion snap-fit device. Other suitable secure features for engagement may be used.

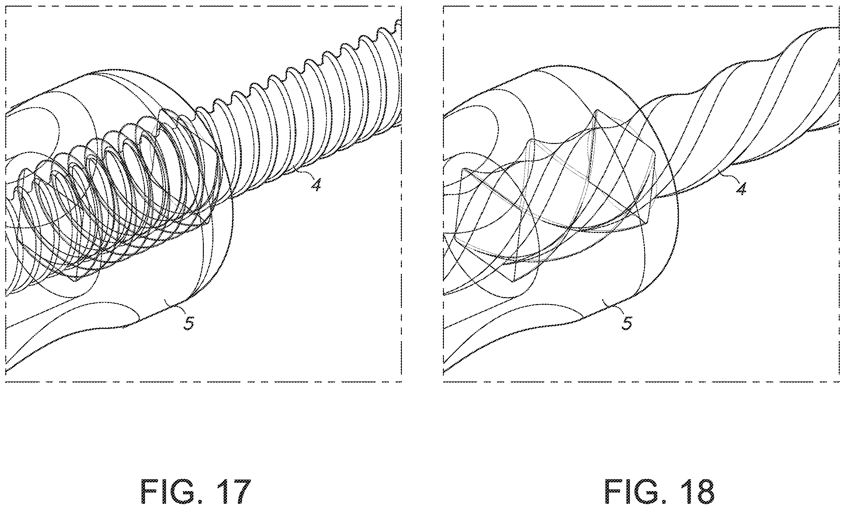

In preferred embodiments, the hinge system 3 is set so as to engage the hinge pin 12 into the connector 11 of the threaded mixing screw 4 when the threaded mixing screw 4 is pushed in the distal direction 10 by handle 2 and the hinge pin 12 contained within handle 2. A threaded screw is mated with a threaded nut contained within the mixer which in turn creates rotation. The amount and speed rotation is controlled by the pitch of the screw 4 and mating nut, as can be seen in figs 17 and 18. Engaging the threaded mixing screw 4, via an opening in connector 11, with the hinge pin 12 causes the hinge system 3 of handle 2 to allow powered rotation of the hinge pin 12, hinge and screw 4 assembly as well as the blade 8 and shaft 7 driven by the downward push of handle 2 in direction 10. Since the hinge pin 12 has been removably connected to the hinge/screw and the blade/shaft are fixed to the hinge pin both will rotate simultaneously as they move in direction 10. The number of blade rotations in direction 10 are directly linked to the screw 4 pitch and distance traveled. Thus, a higher screw pitch will decrease the number of rotations per axial stroke (e.g., in direction 9 or 10), while a lower screw pitch will increase the number of rotations per axial stroke (e.g., in direction 9 or 10).

Once handle 2 has been pushed in direction 10 it can be pulled in opposite direction 9. The action of pulling on handle 2 in direction 9 deactivates the hinge system 3 pulling the screw/hinge with it as well as the blade 8 and shaft 7, however since the hinge pin 12 has been disconnected from the opening in the connector 11, the screw 4 can rotate freely as it moves in direction 9 while the mixing head 8 and shaft 7 do not rotate as they too move in direction 9.

The concept of spin-down and pull-up by using a hinge system 3 has been found to provide significantly improved mixing. After numerous trials using a spin-down/spin-up method, it was discovered that the blades in the mixing head 8 would take the same path through the mixing syringe and either would not mix the dry components at the bottom of the mixing chamber with the viscous gel sitting above it, or it would require significantly more strokes (such as more than 50 or more than 100) before the components would be mixed. The challenge was to adequately disperse and mix powder during the upward movement of the handle to accelerate the mixing process. The creation of the hinge system 3 housed within the handle 1 eliminated any need for switches or levers and becomes automatic and blind to the user, while facilitating effortless mixing in as few as 5 strokes, 10 strokes, or within 20 strokes regardless of the mixture or viscosity.

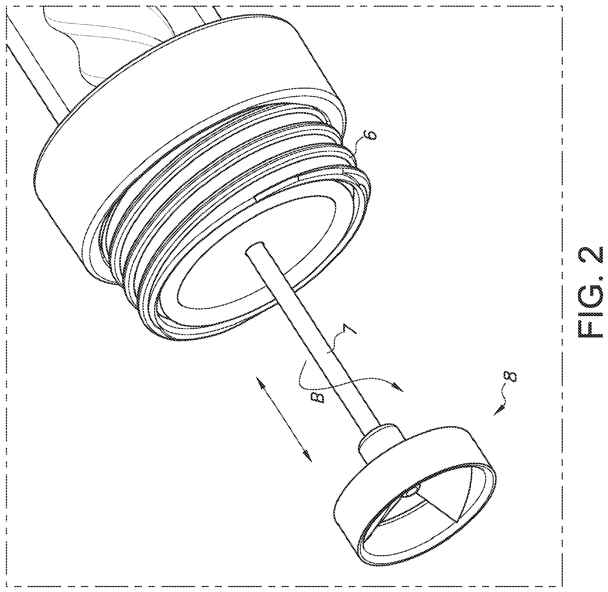

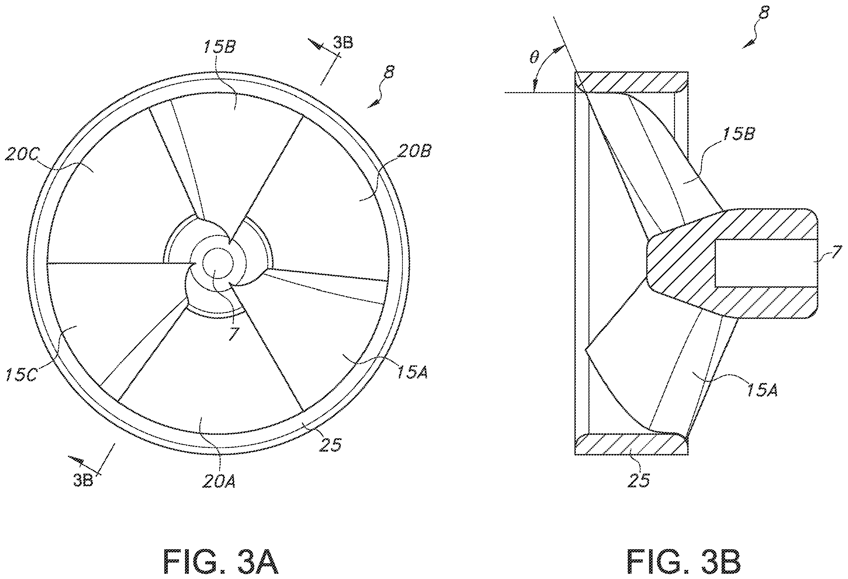

Figures 2, 3A and 3B show a mixing head 8. As can be seen, in this embodiment, the mixing head 8 is secured to the distal end of a rod 7, which is slidably disposed within the mixing body 5, and has an engagement feature 6 at the distal end of the body 5. The line designated "B" shows the rotation of the mixing head 8 as it is moved in the distal direction, for example, if the rod 7 is pushed distally and is allowed to rotate. This embodiment shows the rotation of the device in a clockwise direction, but the mixing head 8 may be moved in a counter clockwise direction if desired. The mixing head 8 includes a plurality of mixing blades 15, spaced apart by gaps 20, all disposed circumferentially between central opening 16 and mixing head cylinder 20. The mixing head 8 is a generally cylindrical device, defined at its outer surface by mixing head blade ring 25. Rod 7 is secured to the mixing head at the central opening 16.

In the embodiment seen in Figures 2-3, the mixing head has three mixing blades 15A, 15B, 15C, separated by three gaps 20A, 20B, 20C. Each blade 15 is expands in size from the central opening 16 to the mixing blade ring 25. As seen in Figure 3B, the mixing blades 15 may have an angle THETA, as defined by the line formed by the central axis A. The mixing blades 15 may be angled from the distal end of the blade ring 25 to the proximal end of the blade ring 25, to aid in mixing. Angle THETA may be from about 45 degrees to about 85 degrees or about 45 to about 67 degrees. The angle may be made to substantially conform to the plunger shape and angle. By conforming the angle, it can scrape materials such as powder and gel from the plunger face for better mixing. By way of example only, the angle of a 100 ml syringe plunger may be approximately 80°.

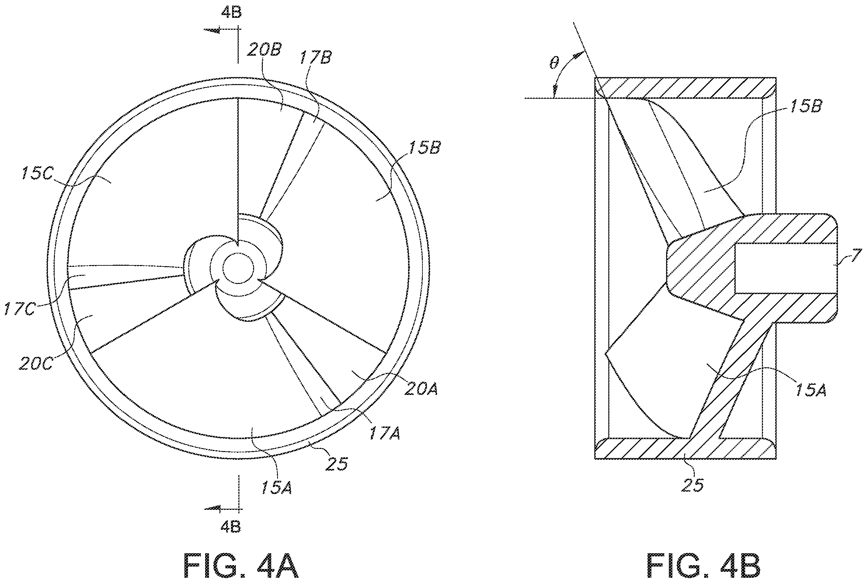

Figures 4A-4B show an alternate mixing head. In this embodiment, there are three mixing blades 15, each separated by gap 20, however, each mixing blade 15 is larger in circumferential size than the embodiment of Figures 2-3. Further, each gap 20 is smaller in circumferential size than in Figures 2-3. The mixing edge of the mixing blades 15 may include an angled region 17, to aid in mixing. In this embodiment, each gap 20 is smaller than each mixing blade 15 as measured by its circumferential size within the blade ring 25.

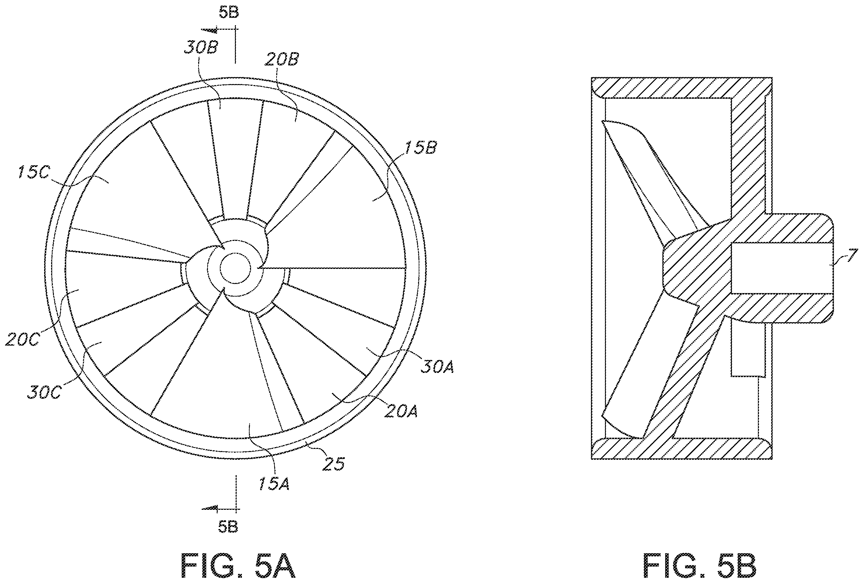

Figures 5A-5B show another mixing head 8. In this embodiment, the mixing head includes three mixing blades 15 and gaps 20, however, an additional spoke 30 is disposed within each gap 20, extending from the central region of the mixing head to the blade ring 25. Spokes 30 may be flat or they may be rounded or shaped in a desired configuration.

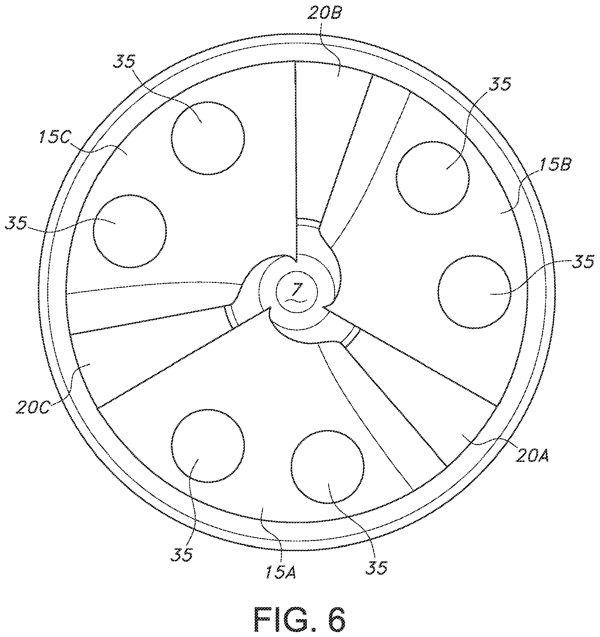

Figure 6 shows another embodiment of a mixing head, which includes three mixing blades 15 and three gaps 20 therebetween. However, this embodiment includes a plurality of holes 35 through each mixing blade 15. There may be at least one hole 35 within each mixing blade 15, where the hole 35 extends through the mixing blade 15 in the axial direction A (seen in Figure 1).

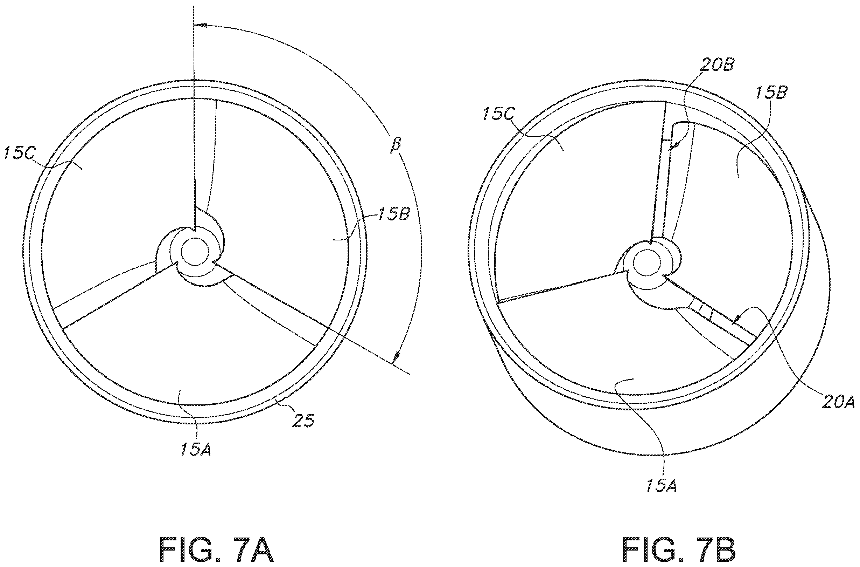

Figures 7A-7B show yet another embodiment of a mixing head, which includes three mixing blades 15 each separated by a gap 20, however, in this embodiment, each mixing blade has a circumferential size BETA, as measured at the blade ring 25. Each blade 15 is sized so as to slightly overlap the adjacent blades 15, and as such from the front view (Figure 7A), the gaps are not noticeable. Each mixing blade 15 in this fashion has a circumferential angle of about 120 degrees, and each blade 15 overlaps an adjacent blade due to helical pitch. Each mixing blade 15 is angled from proximal end of the blade ring 25 to the distal end of the blade ring 25, allowing for mixing of materials between each blade 15.

In embodiments where multiple blades 15 are used, each blade may have a circumferential angle of from about 120 degrees to about 45 degrees, including angles of about 90 degrees, about 66 degrees, or about 60 degrees. The circumferential angle of the blade 15 may depend upon the circumferential angle of the gap 20 between adjacent blades 15. The blades may have any helical pitch, from about 2.54 cm (1.0 inches) to about 0.25 cm (0.10 inches), or from about 1.78 cm (0.70 inches) to about 0.51 cm (0.20 inches), or from about 1.32 cm (0.52 inches) to about 0.64 cm (0.25 inches), or from about 1.02 cm (0.40 inches) to about 0.76 cm (0.30 inches). The helical pitch of the blade 15, in combination with the blade angle, number of blades and circumferential coverage of the blades in the mixing head 8 all contribute to the total open shear area of the mixing head 8. The open shear area may be from 6.5 cm² (0.100 in²) to about 0.0065 cm² (0.001 in²), and may be about 0.52 cm² (0.08 in²), or about 0.42 cm² (0.065 in²), or about 0.32 cm² (0.05 in²), for example in a mixing head capable of fitting in a typical 10 mL syringe. It is contemplated that the open shear area may increase or decrease as the diameter and overall size of the mixing head changes to be suitable for a larger or smaller mixing apparatus (such as a syringe).

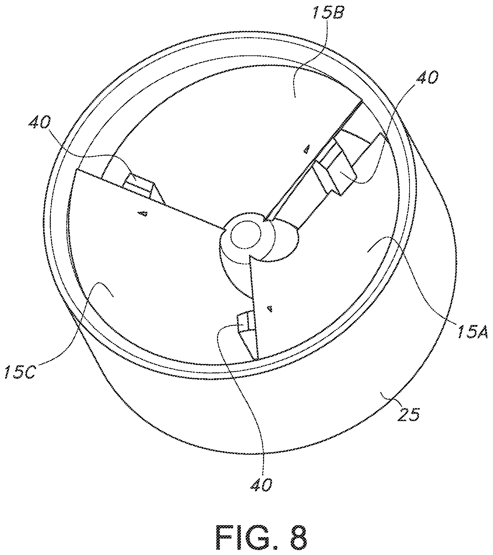

Figure 8 shows another mixing head 8, including three mixing blades 15, but also includes a vertical blade 40 connecting adjacent mixing blades 15 and spanning each gap 20.

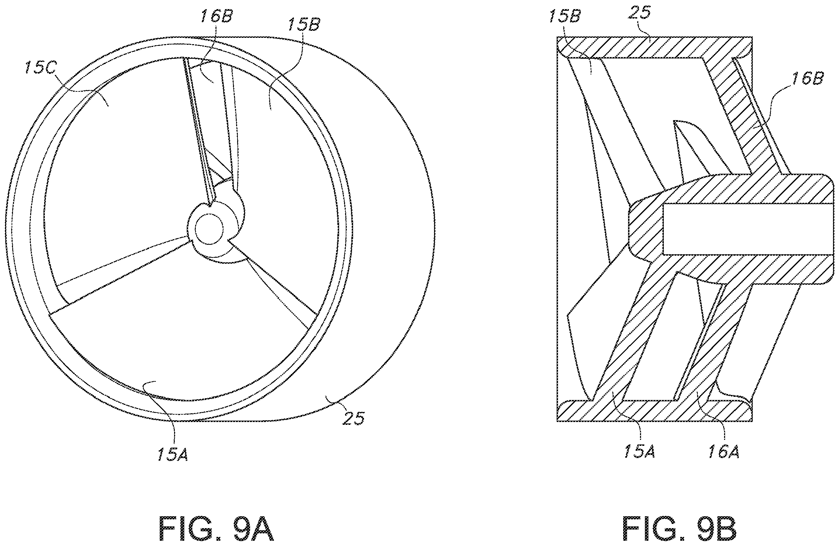

Figures 9A-9B show another mixing head 8, which includes two sets of mixing blades 15, offset by each other along the axial direction A. First set of mixing blades 15 are disposed distally, while second set of mixing blades 15 are offset proximally. Of course, it can be contemplated that the position of the proximal blades and distal blades can be aligned with each other, or off-set from each other within the blade ring 25. There is a gap between each set of mixing blades 15, so as to allow the flow of materials therebetween and allow mixing of materials.

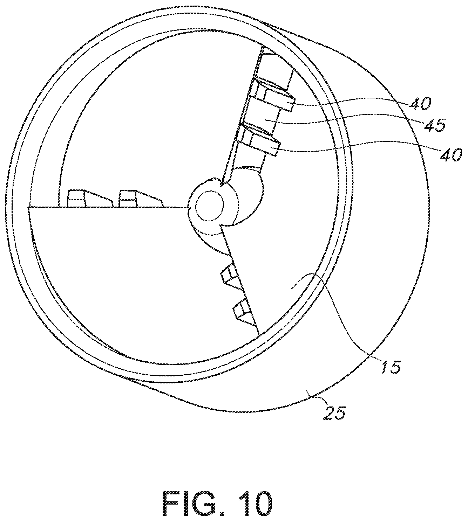

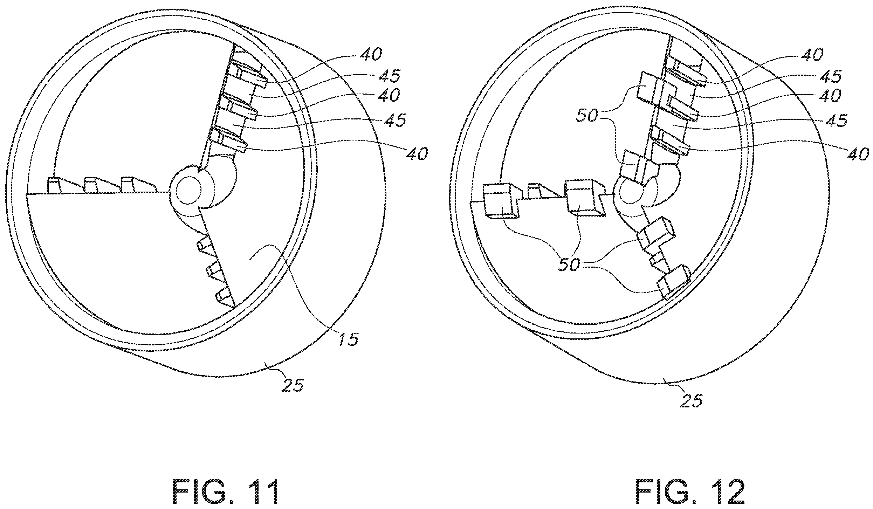

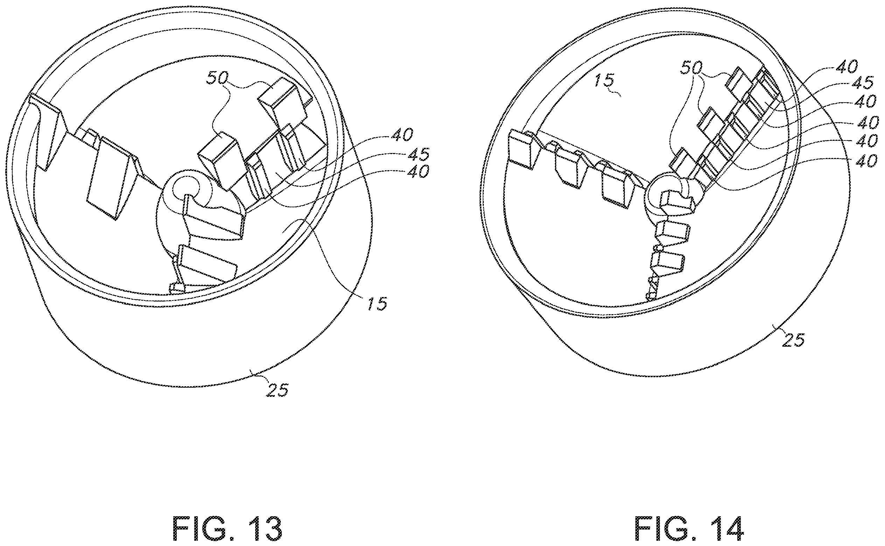

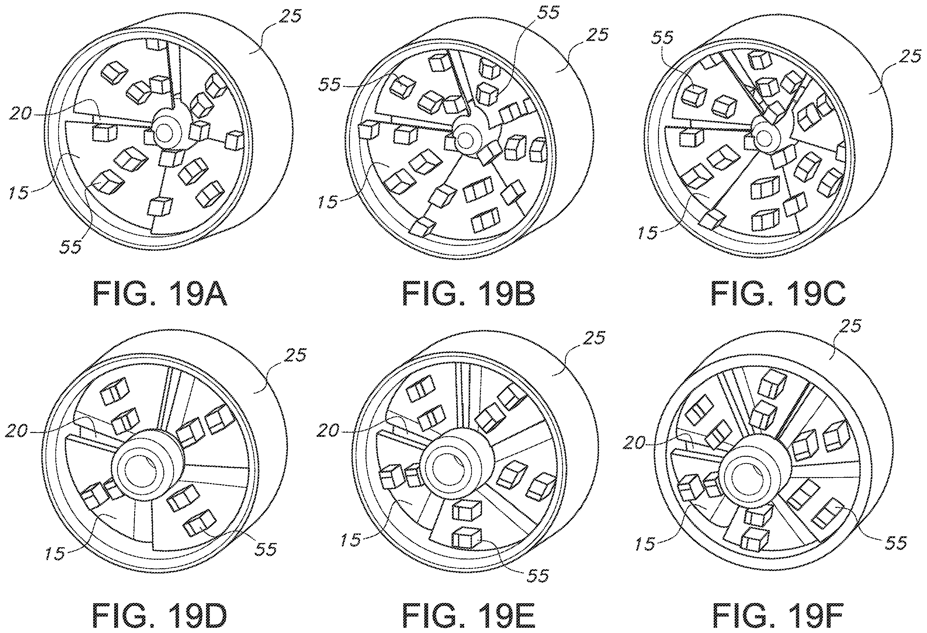

Figures 10 and 11 show a mixing head similar to that of Figure 8, but Figure 10 shows two vertical blades 40 within each gap 20 and connecting adjacent mixing blades 15. A gap 45 is disposed between each vertical blade 40. Figure 11 shows three vertical blades 40 within each gap 20 and connecting adjacent mixing blades 15. Figure 12 shows the mixing head of Figure 11, but further including a plurality of raised blades 50. Raised blades 50 are useful in contacting a syringe piston when in use, effectively scraping material off the surface of syringe piston. Figures 13 and 14 each show a mixing head 8 including two vertical blades 40 connecting adjacent mixing blades 15, with a plurality of more aggressive angled raised blades 50 which are useful in digging into the dry or packed powder on the downward stroke end to disturb the powder and allow the powder to pass through and be located above the blades 15. Being located above the blade allows the powder to be pulled upwards on the return stroke thus aiding in complete and quick mixing. For example, when a raised blade(s) is located above the blade as can be seen in Figure 19, the blade(s) acts as an obstruction, thus forcing the components to be mixed (e.g., gel and powder) around the blade(s) as the blade(s) moves upwards on the return stroke. This aids in achieving complete, efficient and suitable mixing. As can be seen in Figures 13 & 14, protrusions 50 are staggered across the blades diameter, so as to cover some or the entire cross-sectional surface; however the protrusions could also be in alignment with each other.

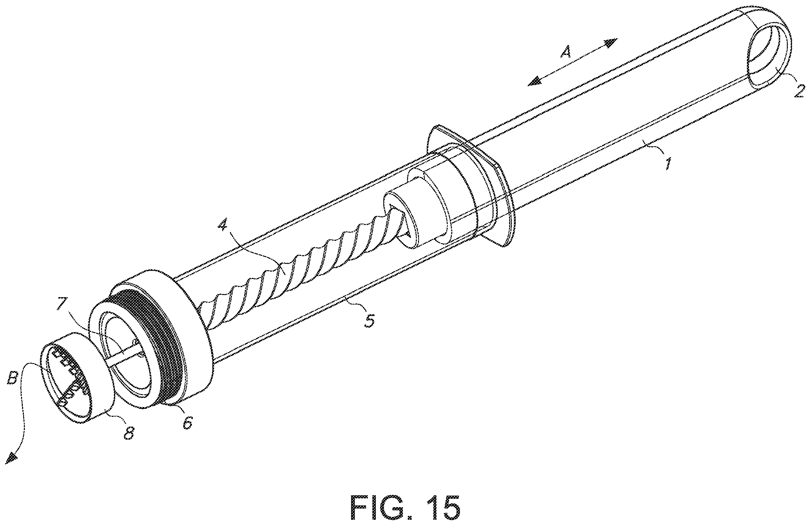

Figure 15 shows an alternate embodiment of a mixing apparatus, which has a modified compact safety plunger handle to guard the user glove or hand from possible engagement with the screw. As with prior disclosures the handle can contain the hinge system 3. The other components, including mixing head 8, rod 7, engagement feature 6, body 5, and threaded mixing screw 4 include those configurations described above. In this embodiment, however, the plunger 1 is elongated, and is fit such that the plunger enters the interior of the body 5 when pushed in the distal direction. An ergonomic holder 2 may be used, including, for example, a finger or thumb engaging recess.

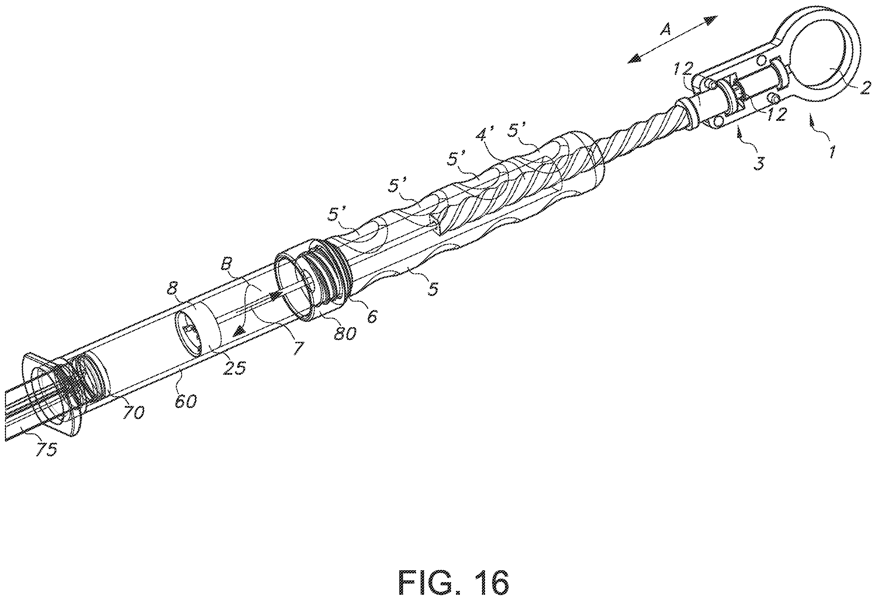

Figure 16 shows an assembled mixing apparatus connected to a syringe, where the plunger is shown in cross-section, and the body 5 is shown as see-through or transparent (note, the body 5 need not be transparent, and may be opaque if desired). As can be seen, in this embodiment, the mixing apparatus is connected to a syringe, such that the distal end of the mixing apparatus (at engagement feature 6) is connected to the dispensing end of the syringe (at syringe engagement feature 80). Any engagement feature may be used, and for example, mixing apparatus engagement feature 6 may be a male threaded region, and syringe engagement feature 80 may be a female threaded region. Other connections are useful, including, for example, friction fit or snap fit. Any standard syringe with a modified engagement feature 80 may be used, and the syringe may include features such as a cylindrical body 60, plunger septum 70 snugly fit within cylindrical body 60, where the plunger septum 70 is connected to a syringe plunger 75. The syringe body 60 may be any size, and may be from about 5 mL in volume to about 100 mL in volume, or it may be 10 mL, 20 mL, 30 mL, 40 mL, or 50 mL.

It is particularly desired that the outer blade ring 25 of the mixing head 8 be sized so as to be axially movable within the syringe body 60, where the diameter of the mixing head 8 is slightly smaller than the inner diameter of the syringe body 60. In some embodiments, the diameter of the mixing head may be equal to or less than 1 mm smaller than the inner diameter of the syringe body 60, and may be equal to or less than 0.1 mm smaller than the inner diameter of the syringe body 60. It is also contemplated that mixing head 8 may have at least one radially extending flexible extension or extensions that are sized and shaped to contact and wipe the interior walls of the syringe upon axial movement of the mixing head 8. Such extensions could be aligned in the direction of travel "A" or within 90 degrees of "A".

When the mixing apparatus and syringe are connected to each other, the mixing head 8 is disposed within the syringe body 60, and is axially movable distally and proximally by pushing or pulling the mixing plunger 1. As can be seen in Figure 16, when pushed distally, the mixing head 8 rotates along rotation path B, but when pulled proximally, the mixing head 8 does not rotate. This may be reversed, whereby the mixing head 8 rotates when pulled proximally but does not rotate when pushed distally.

The threaded mixer screw 4, as described above, is useful in the mixing apparatus. The screw 4 has a series of threads running along its outer surface, from proximal end to distal end.

Figures 17 and 18 show different exemplary pitches of the threads of the mixing screw 4. The pitch of the threads on the screw 4 may be modified to suit its desired purpose, with the understanding that the size, spacing and amount of threads along the screw 4 has an effect on the rotation of the mixing head 8 in use. For example, there may be a 0.64 cm (0.25 inch) pitch as seen in Figure 17, or there may be a 2.54 cm (1.0 inch) pitch as seen in Figure 18 (the pitch may be from about 0.25 to 2.54 cm (0.10 to 1.0 inches), for example). There may be a double helix or triple helix design used, which may increase rotational speed and number of rotations. Other pitches are contemplated by this invention, however one can imagine pitches higher as well as lower keeping in mind that the lower the pitch the harder it may be for the user to accomplish axial movement. Additionally, it has been discovered that complete mixing can be usually achieved in as little as 5 strokes, or 10 strokes, but due to the ease of stroking the mixer and comfort of knowing the mix is homogeneous most mixing may be achieved within 20 strokes. Further it has been found that the range of blade rotations to achieve complete mixing ranges from about 50 to about 150 rotations in a 10ml syringe mixer, but typically between 80 and 120 rotations, or about 100 rotations. Mixing varies not only with the screw pitch, but also on the blade or blades used, as well as the material(s) being mixed. It has also been found the as the mixer volume and cylinder diameter get larger, mixing becomes quicker and easier due to the increase blade rotational speed at the blade ring 25, thus creating increase mixing capacity and if needed shear with blade design.

The number of revolutions may be greatly affected depending upon the screw pitch, since the smaller the screw pitch, the greater the number of rotations per axial stroke. For both 2.54 cm (1") & 1.02 cm (0.4") pitch screws, it was experienced that approximately 120 total rotations was sufficient to provide a suitable and effective mixed composition. That would be, for example, about 20 strokes for a 1.02 cm (0.4") pitch screw and about 50 strokes for a 2.54 cm (1") pitch screw. As can be seen from the chart below, a 1.02 cm (0.4") pitch screw could be rotated 300 times if there are 50 strokes. In some embodiments, the device should be used to mix components such that the mixing head is rotated from about 50 to about 300 rotations, or about 100 to about 200 rotations, or about 120 to about 150 rotations. The number of strokes may vary depending upon the pitch and the axial length of the device. By way of example, using a typical 10ml syringe body of approximately 6.1 cm (2.4") in length, the total number of revolutions (or rotations) of the mixing head for a given number of strokes can be estimated as follows:

| **Strokes** | **Screw pitch /cm (inch)** | | | | | | | | |
|---|---|---|---|---|---|---|---|---|---|
| | **2.54 (1)** | **1.91 (0.75)** | **1.65 (0.65)** | **1.27 (0.5)** | **1.14 (0.45)** | **1.02 (0.4)** | **0.89 (0.35)** | **0.76 (0.3)** | **0.64 (0.25)** |
| **10** | 24 | 32 | 37 | 48 | 53 | 60 | 69 | 80 | 96 |
| **20** | 48 | 64 | 74 | 96 | 107 | 120 | 137 | 160 | 192 |
| **30** | 72 | 96 | 111 | 144 | 160 | 180 | 206 | 240 | 288 |
| **40** | 96 | 128 | 148 | 192 | 213 | 240 | 274 | 320 | 384 |
| **50** | 120 | 160 | 185 | 240 | 267 | 300 | 343 | 400 | 480 |
| **100** | 240 | 320 | 369 | 480 | 533 | 600 | 686 | 800 | 960 |
| **200** | 480 | 640 | 738 | 960 | 1067 | 1200 | 1371 | 1600 | 1920 |

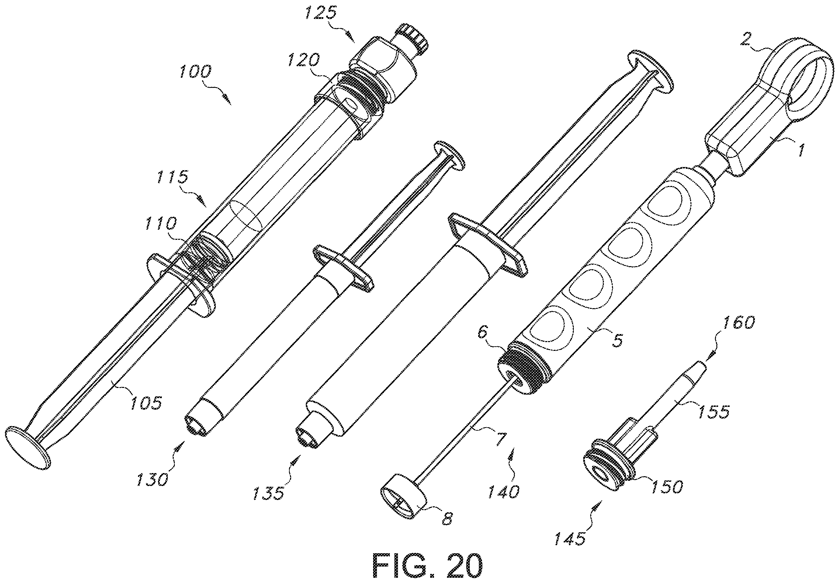

Figures 19A-19F are perspective views of various mixing heads with raised protrusions. As can be seen, in each embodiment, the mixing head includes at least one gap 20, which allows the flow of fluid material through the mixing head during use. Figures 19A-19C show the distal side of the mixing head, while Figures 19D-19F show the proximal surface of the mixing head. Mixing head may include various protrusions or raised teeth 55 on its surface, desirably its distal surface. There may be various configurations of raised protrusions 55 on the surface. It is helpful to include these protrusions 55 on the distal surface, since, during use, the distal surface of the mixing head may come into contact with the plunger septum 70 of the syringe. The protrusions 55 can help to scrape material off of the plunger septum 70 surface during use. Protrusions 55 on either distal or proximal sides may also help agitate the fluid materials within the mixing chamber (e.g., a syringe body 60) and can aid in mixing by causing the material to flow around protrusions 55 or breaking up clumps during the spinning or non spinning direction. As noted above, the mixing head may include one or more outwardly extending flexible protrusions about the outer circumference of the ring 25, which contact the inside walls of the mixing chamber (such as syringe) and wipe the walls upon axial stroke. The present invention also includes a kit, which may include various tools and devices to be used by a user. Figure 20 is a depiction of various components that may be useful in a kit. The kit may include a mixing and dispensing syringe 100, which includes a plunger 105, plunger septum 110, syringe body 115, dispensing end 120, and optional disposable seal cap 125. The seal cap 125 may include a port for injection of materials, such as saline or a gel material and may include a tube extending from the seal cap into the syringe body 115. It has been found that adding of materials to the syringe may be done from the bottom to avoid trapping air which can happen when filling viscous gel from the top. In addition, syringe 100 may be fitted with a feature for restraining the plunger 105 from moving distally during mixing. The syringe body 115 may be generally cylindrical, as explained above. The dispensing end 120 is desirably an open end of the cylindrical body 115, and may include attachment feature, such as threaded regions or snap-fit or friction fit regions. Seal cap 125 may be sized and shaped to be fit onto the dispensing end 120 of the syringe 100. The mixing and dispensing syringe 100 may include a pre-filled material, such as powder material or a gel material. The pre-filled material may include, for example, ORC powder.

A kit may further include pre-filled liquid syringe 130, including a liquid material such as water, saline or other aqueous material-filled syringe. The syringe may be smaller in size than the mixing and dispensing syringe 100, and may include any desired amount of liquid material. The kit may further include a second pre-filled syringe 135, which is pre-filled with a second material, such as a gel or other powder material to be used. This second material may include, for example, a cellulose gel, such as carboxymethyl cellulose. The combination of volume from liquid syringe 130 and second pre-filled syringe 135 and materials in syringe 100 should be equal or less volume than syringe 100 can hold. The kit further includes a mixing apparatus 140, as explained above. The mixing head 8 has a circumference that is sized and shaped to be fit into and through the dispensing end 120 of the mixing syringe 100. The mixing apparatus 140 should also include an engagement feature 6, which is configured to be mated with the dispensing end 120 of the mixing syringe 100. The kit may optionally include a dispenser tip 145, which includes an engagement feature 150, where the engagement feature 150 is substantially similar to the engagement feature 6 of the mixing apparatus 140 and may include a seal to prevent leakage during mixing or dispensing. The dispenser tip 145 may include an interior dispensing lumen 155, which terminates at an orifice 160 at a dispensing end. In use, the dispenser tip 145 may be secured to the dispensing end 120 of the mixing syringe 100 to aid in dispensing of the mixed material. The dispenser tip 145 may include an attachment feature such as a standard luer-lock fitting at the orifice 160 to secure an elongated tube or catheter for delivery to an internal site, such as within a lung or other body region.

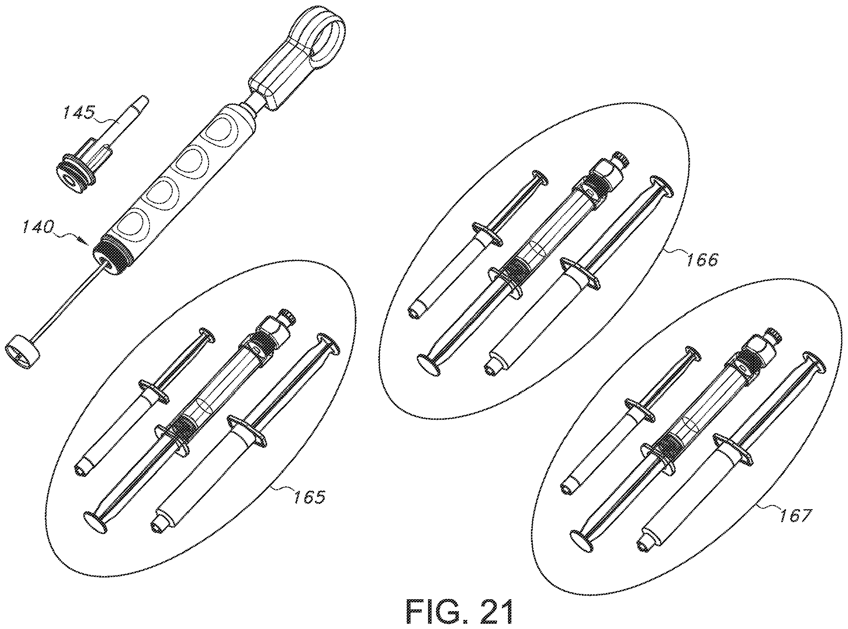

Figure 21 is a depiction of a kit that may be provided, where the kit includes a reusable mixing apparatus 140 and dispenser tip 145, but packages or kits of pre-filled materials 165 are provided to a user. A kit may include a package 165 of materials, or it may include three packages 165, 166, 167, where each package includes a set of pre-filled syringes, including, for example, a pre-filled liquid syringe 130, a pre-filled mixing and dispensing syringe 100, and pre-filled second material containing syringe 135, as described above. These three materials may be sold as an individual kit (e.g., 165, 166, 167), where the mixing apparatus 140 and dispenser tip 145 are reusable or sold as separate devices.

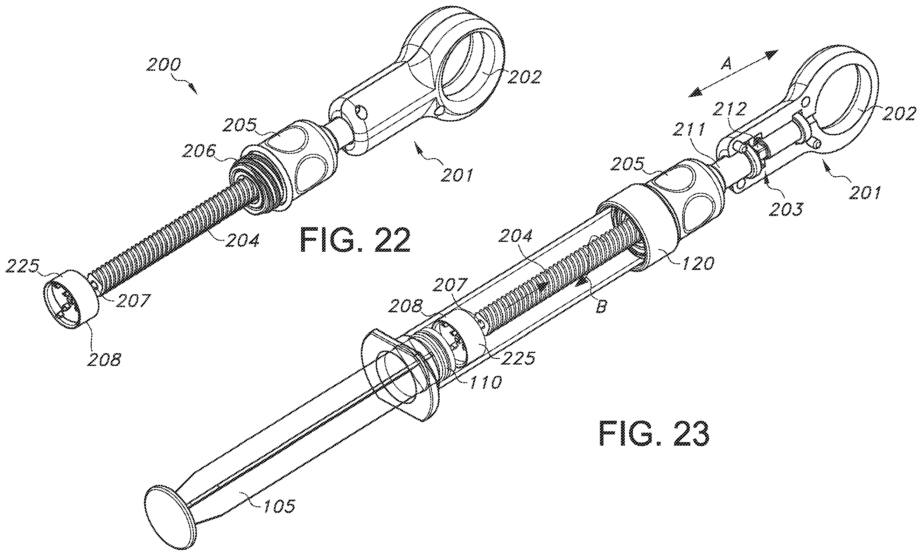

The mixing apparatus may be a compact mixing apparatus 200 where the drive screw enters the material being mixed opposed to earlier embodiment where only the shaft 7 and blade 8 are in contact with the mixture, as seen in Figure 22. Figure 23 shows a compact mixing apparatus 200 in use and as connected to a delivery syringe 100. As can be seen, the compact mixing apparatus 200 includes many of the same components described above, including plunger 201, ergonomic holder 202, hinge system 203 including hinge pin 212, threaded screw 204 with connector 211, and mixing body 205. The compact mixing apparatus 200 may also include a locking or attachment mechanism 206 for securement to the dispensing end of a syringe 100. The compact mixing apparatus 200 also includes a mixing rod 207 terminating in a mixing head 208, which has a generally cylindrical blade ring 225. In the compact mixing apparatus 200, however, the threaded screw 204 is generally disposed outside and more distal than the body 205, and the threaded screw 204 may be placed within the interior body 115 of a syringe 100 when in use. The benefit of a compact mixing apparatus 200 is that it may take less space and be easier to manipulate by a user than the traditional mixing apparatus (e.g., 140) described above, and any of the described embodiments can be of various mixing volumes and mixing volume can be comprised of containers that are short and fat, or long and thin.

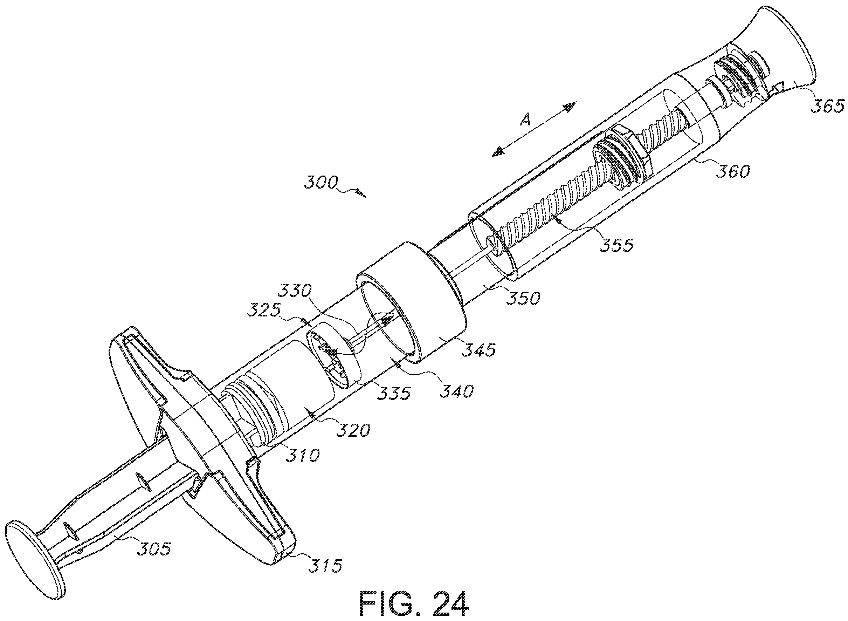

Figure 24 shows another depiction of a compact large (e.g., about 30-50 ml) mixing apparatus with a safety handle and delivery syringe connected to each other as an assembly 300. The general components are the same, including a syringe plunger 305, syringe piston 310, with an optional syringe lock 315 secured to the syringe to prevent the syringe plunger 305 from being removed from the syringe body 340. The syringe may include a pre-filled material 320, such as a powder, or the material 320 may be added prior to use. The assembly 300 further includes a mixing head 325, connected to a mixing rod 330, where the mixing head 325 includes a generally cylindrical outer ring 335. The cylindrical outer body 335 is sized and shaped to be slightly smaller in diameter than the inner surface of the syringe body 340, to effectuate proper and thorough mixing of materials within the syringe body 340. The assembly includes a syringe engagement mechanism 345 and mixing engagement mechanism (not seen), where the engagement mechanisms are to be mated with each other, and may include threads, snap fit, friction fit, or other attachment feature. The assembly 300 further includes a mixer body 350, mixer threaded screw 355 with proximal connector 356, and plunger 360, where the plunger handle 360 may include an ergonomic holding feature 365 as well as may include a hinge system (not shown). Feature 365 includes mating screw features shown but also could include snap or welded ultrasonic posts.

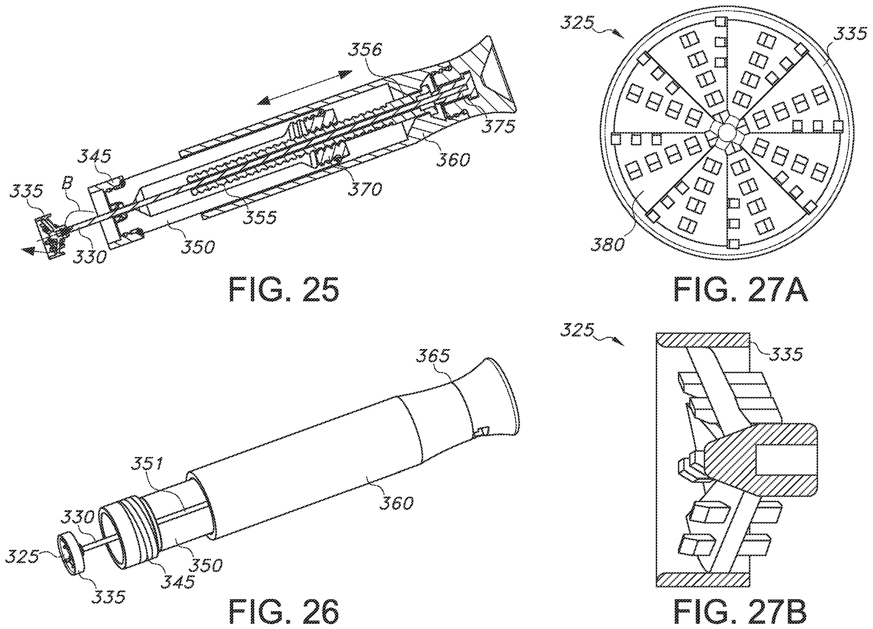

As can be seen in Figure 25, which is a cross-sectional view of the mixing apparatus of Figure 24, the mixing device further includes a nut 370 contained within the mixing body 350 and an anti-rotational rib 351 used to stop rotation of the handle body relative to the mixing body, through which the screw 355 is placed. The device further includes a hinge system 375, as described above to allow rotation of the mixing head 325 in only one direction (e.g., either distally or proximally). Figure 26 is a side view of the mixing apparatus of Figure 24, as not secured to the syringe body. The mixer attachment feature 345 can be seen, which includes a series of threads but any attachment feature is contemplated.

Figure 27 is a front and side view of a mixing head 325, where the mixing head includes a generally conical shaped blade 380 (viewed from the side), and includes a series of holes or pass-through regions as part of the blade. Any mixing heads as described previously may be useful in this embodiment, with any number of blades, blade angle, blade pitch, blade coverage and open shear area, as well as including protrusions or lacking protrusions. For example, a blade configuration may include three blades, each with a blade angle of about 120 degrees, a blade pitch of about 1.32 cm (0.52 inch), with 360 degree blade coverage and an open shear area of about 0.439 cm² (0.068 in²). Another blade configuration may include three blades, each with a blade angle of about 120 degrees, a blade pitch of about 1.07 cm (0.420 inch), with 360 degree blade coverage and an open shear area of about 0.34 cm² (0.052 in²). Another blade configuration may include three blades, each with a blade angle of about 120 degrees, a blade pitch of about 0.81 cm (0.32 inch), with 360 degree blade coverage and an open shear area of about 0.27 cm² (0.042 in²). Another blade configuration may include six blades, each with a blade angle of about 60 degrees, a blade pitch of about 0.64 cm (0.250 inch), with 360 degree blade coverage and an open shear area of about 0.14 cm² (0.022 in²).

Thus, as explained above and as seen in the various Figures, mixing heads may include any number of blades, varying blade angles, pitches, coverage and open shear area. The aforementioned four blade configurations are representative, and it is understood that variation in the specific sizes, angles and open shear area may occur.

Further, and with respect to any of the embodiments described above, the mixing chamber, such as a syringe, may have any size, and may be a 5 mL syringe, 10 mL syringe, 30 mL syringe, 50 mL syringe, 100 mL syringe, or any other size desired. The relative sizes of the mixing components may be modified to fit within any size device and achieve mixing.

The present invention is also directed to a method of mixing at least two materials, including gels, powders, liquids, and combinations thereof, however it could also be used to re-mix a pre-mixed solution prior to use if so desired. In one aspect, there is a method of mixing a gel and a powder. Optionally, a liquid material, such as water or saline, may be mixed in combination with the gel and/or the powder. Although the order of mixing may be reversed, in one embodiment, water or saline, or another thinning material, may be added to a powder component first in order to wet the powder to aid in mixing. Water or saline or other thinning material may be added to any other additional materials if desired.

In one method, a syringe is filled with the desired materials to be mixed. The syringe may be pre-filled with one or more materials, or the materials may be filled prior to use. For example, the syringe may be filled with a gel component, such as a cellulose, a powder component, such as ORC, and may include a liquid material such as saline or water to aid in mixing. The syringe piston may be inserted into the syringe and may be held in place via a locking feature during mixing.

A mixing apparatus, as described above, may be secured to the dispensing end of the syringe via locking feature, such as mated threads, snap fit, or friction fit. In this configuration, a mixing head is disposed within the body of the syringe, where the outer surface of the mixing head is the same diameter or slightly smaller in diameter than the inner circumference of the syringe body. A plunger is used to push and pull the mixing head axially, such that it moves from a distal direction to a proximal direction, and back in the distal direction a desired number of times. Through the use of a hitch mechanism, the mixing head rotates in only one direction, e.g., either the distal direction or the proximal direction, and remains un-rotated in the opposite direction (spin-down/pull-up).

As the mixing head is moved in the rotating direction, the various materials within the syringe body are rotated, agitated, and mixed by being passed through the blade gaps or holes in the mixing head. When the mixing head is moved in the opposite direction, it does not rotate, and therefore has the effect of pulling the various materials by vacuum and force, through the blade gaps and holes in the mixing head. The mixing head is moved a sufficient number of times, which may be from about 5 to about 50 series of pushing and pulling (where a push-and-pull counts as one "series"), or may be from about 10 to about 20 series. Once sufficient mixing has been achieved, which results in a mixed composition that is useful for dispensing or delivery, the mixing head may be stopped in its position, such as a proximal position. At this point, the composition is considered sufficiently mixed, and no further strokes of the mixing head are required.

After sufficient mixing to form a sufficiently mixed composition, the mixing device may be removed from the syringe. If bulk mixed material is not going to be used, either a dispensing tip or a cap may be secured to the dispensing end of the syringe. If a cap is secured, then the syringe with mixture may be stored for a desired length of time, at which time a dispensing tip may be secured to the dispensing end of the syringe. The user aligns the dispensing tip (or an elongated tube secured to a dispensing tip) with a target site, and depresses the plunger into the syringe, either through pushing or via a screw-type mechanism. This ejects the mixture through the dispenser and to the target site. If required, the mixer can be re-engaged to the syringe for additional mixing, for example, if the mixed composition has become too viscous after sitting.

The present invention, including the devices and assemblies described above, as well as the method of mixing, provides a number of benefits. In addition to manufacturing benefits, there are also provided ergonomic mixing benefits during delivery when expressing thick gels or mixtures through thin delivery devices, such as thin tubes used during laparoscopic surgery or when inserted into a bodily region such as a lung. The present invention includes a two-piece design (a delivery syringe and mixing device), which allows the use of standard syringes (for example, 10 mL syringes) to be utilized. Use of a syringe lock is useful to prevent the syringe plunger from being expelled during mixing, and then the same syringe body that was used for mixing is used to deliver the mixed composition with ease. This is because some standard syringes have small flanges, making it quite difficult to express viscous compositions. Thus, even if the syringe is a standard syringe that is not modified prior to use, a syringe lock as described herein may be applied to the standard syringe to aid in mixing and delivery of the mixed composition.

If desired, a pressurized gas can be introduced to the syringe body after the mixing has been completed, either prior to or during delivery of the mixed composition. Pressurizing the mixed composition allows for the composition to be sprayed from the syringe. In this fashion, it is useful if the mixture is thoroughly mixed, so that a spray nozzle does not get clogged with unmixed components, such as powder.

The water content of the mixture may be modified as desired to allow for a suitable thickness and water content of the mixture. For example, decreasing the water content of the gel by approximately two ml (e.g., a 4.3% by weight CMC vs. a 3.5% by weight CMC gel) can allow for a thicker, higher viscosity CMC gel which is actually more difficult to mix with a dry powder. However, by then reintroducing water or other fluid directly into the dry ORC powder at the time of mixing, the gel has a better chance of homogeneously mixing with the now-wet ORC powder and then rejoining the gel concentration to achieve a final mixed gel viscosity. This method of using lower water content gel, and introducing water or other liquid (such as saline) may be useful, and is best achieved by using the mixing system and mixing blades described above so as to create appropriate shear.

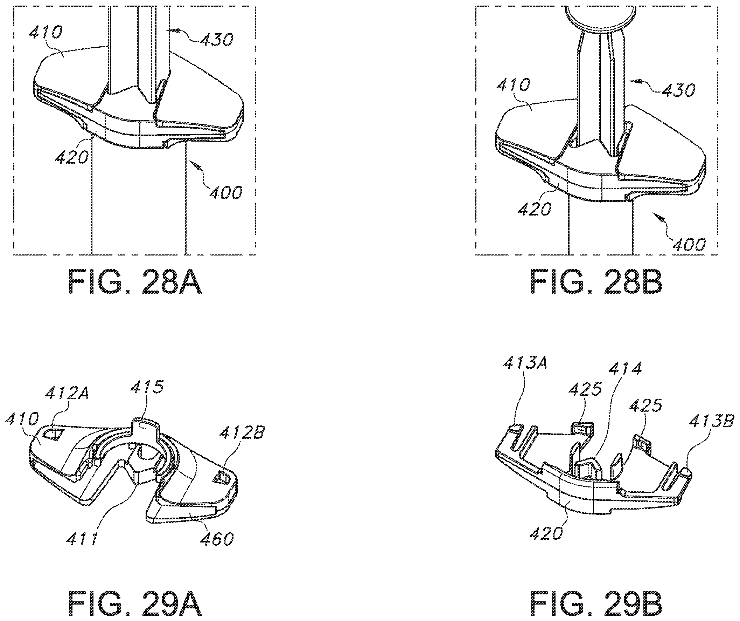

An additional element of the present invention, which may be useful in the assembly described above, is a syringe locking mechanism. With reference to Figures 28-34, the syringe locking system is described.

After limited success attempting various mixing methods (using powder and gel mixing), it was believed that if mixing of powders and gel was to be achieved it would be limited to at most a 5-10 mL or 10-15 mL syringe, due to force issues pushing a syringe plunger during mixing. To address these force issues, a novel two piece custom syringe plunger locking grip is provided. The syringe plunger locking mechanism provides multiple functions, including providing an ergonomic holder during mixing as well as serving as a lock that retains the syringe plunger within the syringe body during mixing. The lock also aids in a sterilization process if the gel is steam sterilized while in the syringe, to prevent expelling the gel due to the plunger being pushed out of the syringe body due to an increase of pressure inside the syringe.

An exemplary syringe locking mechanism 400 includes two components, a first component 410 and second component 420, where the first component 410 and second component 420 are shaped and sized to surround a syringe plunger 430 and flange 440 of a syringe body 450, where the two components (410, 420) are secured to each other. Each component 410, 420 includes a central opening sized and shaped to fit a syringe plunger 430 as well as a syringe body flange 440.

Figures 28A and 28B show perspective views of a syringe lock system, with first component 410 and second component 420 secured to each other. Figure 28A shows a lock assembly 400 secured to a 20 mL syringe (e.g., a larger size syringe), and Figure 28B shows the lock assembly 400 secured to a smaller syringe, such as a 10 mL syringe. Figure 29A shows of the first component 410. The first component 410 may include a sizing tab 415, which may be removed when used on larger syringe bodies, such as 20 mL syringes. Sizing tab 415 allows for the locking assembly 400 to be used on various sized syringes. As can be seen, there is a flange holder 460 in the first component 410, into which a syringe flange 440 can be inserted for secure locking. In use, second component 420 may first house the proximal side of flange 440, and first component 410 may then be slid over second component 420 by means of holder 460, thus securing both the distal side of flange 440 and the proximal top of second component 420. Figure 29B shows the second component 420, which is sized and shaped to mate with first component 410. Second component 420 includes flange positioning tabs 425, which are designed to be inserted into locking apertures 422 of first component 410, and thus provide stability.

The device includes a central "V" shaped protrusion 411 shown in Figure 29A (referred to as the "central V"), as well as the three central protrusions (collectively 414) shown in Figure 29B (referred to as the "three central protrusions"). The central V 411 and the three central protrusions 414 oppose each other, which keeps the plunger from rotating. Similarly the three central protrusions 414 keep the plunger physically located in the syringe as the three central protrusions 414 contact the distal end of the plunger. This ensures that the rubber seals of the plunger remain in the syringe barrel, since the distal most end of the syringe barrel has a larger diameter than the barrel proximal to that area. The two flexible arms 413A, 413B on the outer edge of Figure 29B include locking tabs which engage with two openings 412A, 412B shown in Figure 29A. Flexible arm 413A engages with opening 412A, holding it in place, while flexible arm 413B engages with opening 412B, holding it in place. Once the locking arms 413A/B engage openings 412A/B, respectively, first component 410 and second component 420 are securely located and locked together.

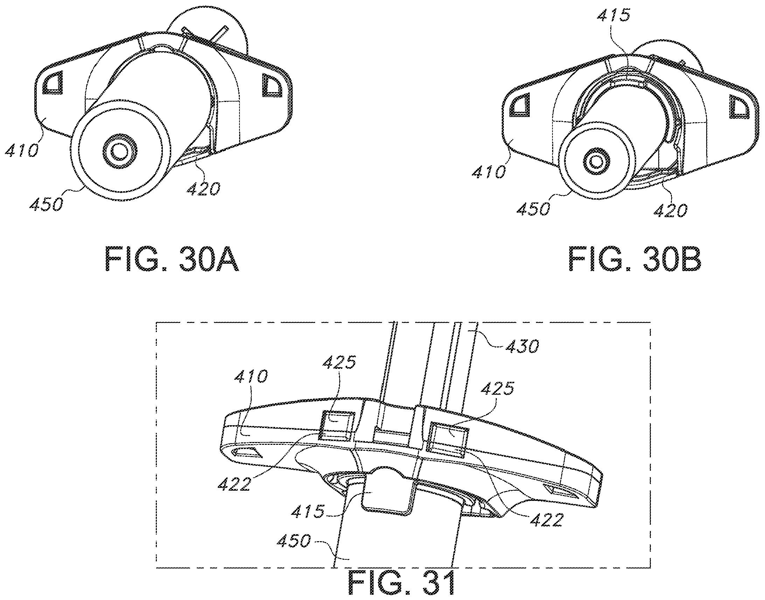

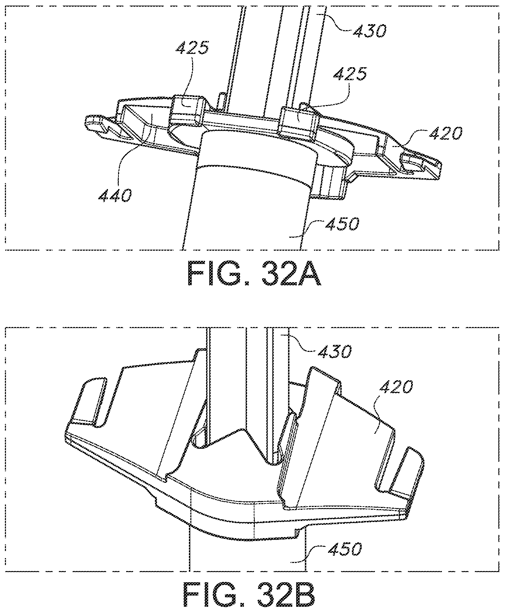

Figures 30A and 30B show the opposing perspective views of the syringe lock assembly 400 of Figures 28A and 28B, respectively. As can be seen, a syringe is contained within the open central region of first component 410 and second component 420. Figure 31 shows the assembly 400 with first component 410 and second component 420 secured to each other, such as through the insertion of flange positioning tabs 425 into open locking apertures 422 of the first component 410. As seen in Figure 31, which is an opposite side view of the assembled syringe lock of Figure 28B, the sizing tab 415 remains secured to the assembly, since the syringe is a 10 mL syringe, but if a larger sized syringe was used, the sizing tab 415 could be removed. Figures 32A-32B show various views of the second component 420 located onto a syringe flange 440. In use, a second component 420 would be positioned over the flange 440, such that the flange 440 would be inserted into the flange holder pocket in the second component 420, and the flange positioning tabs 425 securing the syringe flange edge. First component 410 is then slid over second component 420 and flange 440, such that flange positioning tabs 425 engage locking apertures 422 until the locking tabs on the flexible arms 413A, 413B, are placed into openings 412A, 412B, respectively. This sufficiently engages first component 410 and second component 420, thereby securing flange 440.

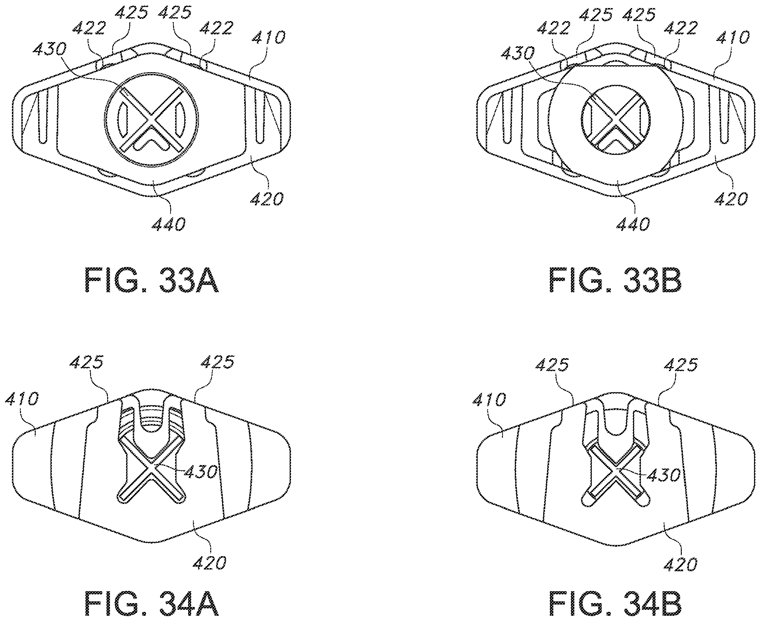

Figures 33A and 33B show cross-sectional views from the top of a syringe lock system, while Figures 34A and 34B show cross-sectional views from the bottom of a syringe lock system. Figures 33A and 34A are syringe lock systems secured to a larger sized syringe (e.g., 20 mL), while Figures 33B and 34B are syringe lock systems secured to a smaller sized syringe (e.g., 10 mL), where the larger sized syringe and the smaller sized syringe include different flange designs. The use of two separate flange designs and two separate syringe sizes confirms that the syringe lock system can be used in varying syringe shapes and sizes, with varying flange configurations. As can be seen, the flange 440 is contained within the flange holder 460 of the first component 410 and second component 420, and the plunger 430 is contained within the open central region of the assembly 400.

The locking assembly 400 prevents the plunger 430 from being completely removed from the syringe body 450 or from being forced to rotate by the mixer blade. The locking assembly 400 is secured to the syringe body 450 by attachment to the flange 440. The plunger 430 is capable of being moved axially through the interior of the body 450, but is prevented from being removed from the body 450 due to the blocking plates of the locking assembly 400. During mixing, force and pressure is generated within the body 450, and the locking assembly 400 prevents unintentional removal of the plunger 430 from the syringe body 450.

### Example

A 10 ml mixer capable of spinning down (distally) and pulling up (proximally) was compared to a mixer capable of spinning down and up. Three different mixing heads, each having a unique blade configuration were tested. Blade 1 included a mixing head having six blades at a 0.64 cm (0.25 inch) pitch, where each blade is 60 degrees, with a number of raised protrusions on its blade surface, having an open shear area between the blades of about 0.142 cm (0.022 in²). Blade 2 included a mixing head having three blades, each blade having a 120 degree circumferential angle and a helical pitch of about 0.81 cm (0.32 inch), having an open shear area between the blades of about 0.270 cm² (0.042 in²). Blade 3 included three blades each having a circumferential angle of about 97 degrees, having an open shear area between the blades of about 0.613 cm (0.095 in²) and a pitch of about 1.83 cm (0.72"). In addition, multiple screw pitches were compared in testing each mixing head, one mixer had a 1.02 cm (0.4 inch) pitch screw and one had a 2.54 cm (1.0 inch) pitch screw.

The composition mixed in each sample included about 8 ml of 4.5% 250 kD medium weight carboxymethyl cellulose, about 2 ml of saline, and about 1 gram of ORC powder with a 4:1 length to diameter aspect ratio. The mixture was expressed as a blob from a syringe, and
testing was conducted using Instron testing for expression forces, which are related to viscosity. The method of characterizing complete mixing was done with visual inspection using a light box to confirm consistency of the mix throughout the syringe.

The results of the testing are set forth in the tables below:

| Blade 1 | | | | |
|---|---|---|---|---|
| Open Shear Area - 0.142 cm² (0.022 in²) | | | | |
| Mixer strokes needed to have complete mixing | | | | |
| Screw Pitch | Spin Down-Pull Up design | Spin Down-Spin Up Design | Percent Increase | Percent Decrease |
| Strokes @ 1.02 cm (0.4") | 25 | 40 | 60% | 37% |
| Strokes @ 2.54 cm (1.0") | 25 | 60 | 140% | 58% |

| Blade 2 | | | | |
|---|---|---|---|---|
| Open Shear Area - 0.271 cm² (0.042 in²) | | | | |
| Mixer strokes needed to have complete mixing | | | | |
| Screw Pitch | Spin Down-Pull Up design | Spin Down-Spin Up Design | Percent Increase | Percent Decrease |
| Strokes @ 1.02 cm (0.4") | 20 | 50 | 150% | 60% |
| Strokes @ 2.54 cm (1.0") | 20 | 55 | 175% | 65% |

| Blade 3 | | | | |
|---|---|---|---|---|
| Open Shear Area - 0.612 cm² (0.095 in²) | | | | |
| Mixer strokes needed to have complete mixing | | | | |
| Screw Pitch | Spin Down-Pull Up design | Spin Down-Spin Up Design | Percent Increase | Percent Decrease |
| Strokes @ 1.02 cm (0.4") | 27 | 90 | 235% | 70% |
| Strokes @ 2.54 cm (1.0") | 30 | 87 | 190% | 57% |

As can be seen, for Blade 1, there was a 37% reduction in number of strokes in the spin-down/pull up as compared to spin down/spin up for a 1.02 cm (0.4 inch) pitch (or, put another way, a 60% increase in spin down/spin up compared to spin down/pull up), and a 58% reduction for a 2.54 cm (1.0 inch) pitch (140% increase for spin down/spin up). For Blade 2, there was a 60%
reduction in number of strokes in the spin-down/pull up as compared to spin down/spin up for a 1.02 cm (0.4 inch) pitch (a 150% increase for spin down/spin up), and a 65% reduction for a 2.54 cm (1.0 inch) pitch (175% increase for spin down/spin up). This blade type appeared to require the least amount of strokes and also provided the highest resulting viscosity. Finally, for Blade 3, there was a 70% reduction in number of strokes in the spin-down/pull up as compared to spin down/spin up for a 1.02 cm (0.4 inch) pitch (235% increase for spin down/spin up), and a 57% reduction for a 2.54 cm (1.0 inch) pitch (190% increase for spin down/spin up).

As can be seen, regardless of blade type, the use of the spin down/pull up configuration provided significant benefit as compared to a mixer that spins in both the up and down directions. In addition, for the spin down/pull up design, there was an increased benefit regardless of screw pitch, as compared to spin down/spin up. The detailed design and concept described above may be scaled to any desired size syringe or range of syringe sizes, and as noted above, different blade configurations may be used.

It was found that the device using a spin down/pull up design resulted in about 37-70% lesser required strokes for full mixing across different blade designs and different pitches with a 1.02 cm (0.4 inch) pitch screw. The Spin down/pull up syringe design resulted in about 57% to 65% lesser required strokes for full mixing across different blade designs and different pitches with a 2.54 cm (1.0 inch) pitch screw. The spin down/spin up design was seen to become less effective as the blade open shear area was increased. The spin down/spin up design is believed to be less effective than the spin down/pull up design, since the blade takes a similar path through the gel in both the up and down directions. By contrast, the spin down/pull up configuration disturbs the gel by forcing it to move through the shear areas of a non-rotating blade in the pull up movement.

It is believed that a larger syringe mixer (such as a 50 ml syringe mixer) will mix as quick or even quicker than a 10 ml syringe mixer given the same blade and screw pitch, due to increased blade diameter having a faster circumferential speed, thus increasing shear and potentially decreasing mixing time.

The testing conducted showed less clogging and jamming, and it was found that the force to operate the present syringe mixer is low, and does not increase as syringe volumes increase. Further, while traditional syringe to syringe mixing of powders and gels generally necessitates smaller and consistent sized particles, the inventive mixing apparatus allows mixing of powders and gels that is not limited to finely ground powders, does not need
consistent sized particles, and is not operator dependent. Therefore, the inventive mixer provides an easier, more effective mixer, with less force required and less risk for operator error.

## Claims

1. A mixing apparatus comprising:
i. a mixing chamber (60) having a first end and second end, where said first end is open;
ii. a mixing device secured to said first end, said mixing device comprising a plunger (1), a rod (7), a mixing head (8), and a device (3) to control spin of the mixing head, wherein said mixing head is inserted at least partially into the mixing chamber, wherein said device to control spin of the mixing head is configured to cause the mixing head to spin when the plunger is moved in a first axial direction (10), and to cause the mixing head to avoid spinning when the plunger is moved in a second axial direction (9);
wherein said mixing chamber is the body of a first syringe, and said first syringe comprises a plunger (75) at a second end:
**characterized in that** the mixing head (8) has a plurality of angled blades (15),
and said mixing head includes a plurality of axially extending protrusions (50) on at least one surface of at least one angled blade on said mixing head.

2. The apparatus of claim 1, further comprising a dispensing tip (145) capable of being secured to said first end of said mixing chamber.

3. The apparatus of claim 1, wherein said mixing head includes an outer ring (25) having an outer wall, and said outer ring includes at least one radially extending protrusion extending from the outer wall of the outer ring.

4. A method of mixing two components comprising:
i. having a first component and a second component into a mixing chamber (60) having a first end and second end, where said first end is open;
ii. securing a mixing device to said first end, said mixing device comprising a plunger (1), a rod (7), a mixing head (8), and a device (3) to control spin of the mixing head, wherein said mixing head is inserted at least partially into the mixing chamber; and
iii. moving said plunger in a first axial direction (10) and in a second axial direction (9) at least one time each, wherein said device to control spin of the mixing head causes the mixing head to spin when the plunger is moved in a first axial direction, and causes the mixing head to avoid spinning when the plunger is moved in a second axial direction;
wherein said mixing chamber is the body of a first syringe, and said first syringe comprises a plunger (75) at a second end; **characterized in that** said mixing head (8) has a plurality of angled blades (15), and said mixing head includes a plurality of axially extending protrusions (50) on at least one surface of at least one angled blade on said mixing head.

5. The method of claim 4, further comprising a dispensing tip (145) capable of being secured to said first end of said mixing chamber.

6. The method of claim 4, wherein said mixing head includes an outer ring (25) having an outer wall, and said outer ring includes at least one radially extending protrusion extending from the outer wall of the outer ring.

7. A kit comprising:
i. a mixing chamber (60) having a first end and second end, where said first end is open, wherein said mixing chamber is the body of a first syringe, and said first syringe comprises a plunger (75) at a second end;
ii. a mixing device capable of being secured to said first end, said mixing device comprising a plunger (1), a rod (7), a mixing head (8), and a device (3) to control spin of the mixing head, wherein said mixing head is inserted at least partially into the mixing chamber, wherein said device to control spin of the mixing head is configured to cause the mixing head to spin when the plunger is moved in a first axial direction (10), and to cause the mixing head to avoid spinning when the plunger is moved in a second axial direction (9);
iii. a first material to be mixed; and
iv. a second material to be mixed,
**characterized in that** said mixing head (8) has a plurality of angled blades (15), and said mixing head (8) includes a plurality of axially extending protrusions (50) on at least one angled blade on said mixing head.

8. The kit of claim 7, wherein said first material is contained within said mixing chamber.

9. The kit of claim 7, wherein the second material to be mixed is provided in a separate container.

10. The kit of claim 7, further comprising a third material to be mixed.

11. The kit of claim 7, further comprising a dispensing tip (145) capable of being secured to said first end of said mixing chamber.

12. The kit of claim 7, wherein said mixing head includes an outer ring (25) having an outer wall, and said outer ring includes at least one radially extending protrusion extending from the outer wall of the outer ring.

## Patentansprüche

1. Mischvorrichtung, umfassend:
i. eine Mischkammer (60), die ein erstes Ende und ein zweites Ende aufweist, wobei das erste Ende offen ist;
ii. eine Mischeinrichtung, die an dem ersten Ende fixiert ist, wobei die Mischeinrichtung einen Kolben (1), eine Stange (7), einen Mischkopf (8) und eine Einrichtung (3) zum Steuern einer Drehung des Mischkopfs umfasst, wobei der Mischkopf zumindest teilweise in der Mischkammer eingeführt ist, wobei die Einrichtung zum Steuern der Drehung des Mischkopfs dazu ausgebildet ist, zu bewirken, dass sich der Mischkopf dreht, wenn der Kolben in eine erste Axialrichtung (10) bewegt wird, und zu bewirken, dass der Mischkopf ein Drehen vermeidet, wenn der Kolben in eine zweite Axialrichtung (9) bewegt wird,
wobei die Mischkammer der Körper einer ersten Spritze ist und die erste Spritze einen Kolben (75) an einem zweiten Ende umfasst;
**dadurch gekennzeichnet, dass** der Mischkopf (8) mehrere abgewinkelte Flügel (15) aufweist,
und der Mischkopf mehrere sich axial erstreckende Vorsprünge (50) an mindestens einer Fläche mindestens eines abgewinkelten Flügels am Mischkopf enthält.

2. Vorrichtung nach Anspruch 1, ferner umfassend eine Ausgabespitze (145), die am ersten Ende der Mischkammer fixiert werden kann.

3. Vorrichtung nach Anspruch 1, wobei der Mischkopf einen äußeren Ring (25) enthält, der eine Außenwand aufweist, und der äußere Ring mindestens einen sich radial erstreckenden Vorsprung enthält, der sich von der Außenwand des äußeren Rings erstreckt.

4. Verfahren zum Mischen von zwei Komponenten, umfassend:
i. Vorsehen einer ersten Komponente und einer zweiten Komponente in einer Mischkammer (60), die ein erstes Ende und ein zweites Ende aufweist, wobei das erste Ende offen ist;
ii. Fixieren einer Mischeinrichtung an dem ersten Ende, wobei die Mischeinrichtung einen Kolben (1), eine Stange (7), einen Mischkopf (8) und eine Einrichtung (3) zum Steuern der Drehung des Mischkopfs umfasst, wobei der Mischkopf zumindest teilweise in der Mischkammer eingeführt ist; und
iii. Bewegen des Kolbens in eine erste Axialrichtung (10) und in eine zweite Axialrichtung (9), mindestens jeweils einmal, wobei die Einrichtung zum Steuern der Drehung des Mischkopfs bewirkt, dass sich der Mischkopf dreht, wenn der Kolben in eine erste Axialrichtung bewegt wird, und bewirkt, dass der Mischkopf ein Drehen vermeidet, wenn der Kolben in eine zweite Axialrichtung bewegt wird,
wobei die Mischkammer der Körper einer ersten Spritze ist und die erste Spritze einen Kolben (75) an einem zweiten Ende umfasst;
**dadurch gekennzeichnet, dass** der Mischkopf (8) mehrere abgewinkelte Flügel (15) aufweist,
und der Mischkopf mehrere sich axial erstreckende Vorsprünge (50) an mindestens einer Fläche mindestens eines abgewinkelten Flügels am Mischkopf enthält.

5. Verfahren nach Anspruch 4, ferner umfassend eine Ausgabespitze (145), die am ersten Ende der Mischkammer fixiert werden kann.

6. Verfahren nach Anspruch 4, wobei der Mischkopf einen äußeren Ring (25) enthält, der eine Außenwand aufweist, und der äußere Ring mindestens einen sich radial erstreckenden Vorsprung enthält, der sich von der Außenwand des äußeren Rings erstreckt.

7. Kit, umfassend:
i. eine Mischkammer (60), die ein erstes Ende und ein zweites Ende aufweist, wobei das erste Ende offen ist; wobei die Mischkammer der Körper einer ersten Spritze ist und die Spritze einen Kolben (75) an einem zweiten Ende umfasst;
ii. eine Mischeinrichtung, die an dem ersten Ende fixiert ist, wobei die Mischeinrichtung einen Kolben (1), eine Stange (7), einen Mischkopf (8) und eine Einrichtung (3) zum Steuern einer Drehung des Mischkopfs umfasst, wobei der Mischkopf zumindest teilweise in der Mischkammer eingeführt ist, wobei die Einrichtung zum Steuern der Drehung des Mischkopfs dazu ausgebildet ist, zu bewirken, dass sich der Mischkopf dreht, wenn der Kolben in eine erste Axialrichtung (10) bewegt wird, und zu bewirken, dass der Mischkopf ein Drehen vermeidet, wenn der Kolben in eine zweite Axialrichtung (9) bewegt wird,
iii. ein zu mischendes erstes Material; und
iv. ein zu mischendes zweites Material,
**dadurch gekennzeichnet, dass** der Mischkopf (8) mehrere abgewinkelte Flügel (15) aufweist und der Mischkopf (8) mehrere sich axial erstreckende Vorsprünge (50) an mindestens einem abgewinkelten Flügel am Mischkopf enthält.

8. Kit nach Anspruch 7, wobei das erste Material in der Mischkammer enthalten ist.

9. Kit nach Anspruch 7, wobei das zu mischende zweite Material in einem getrennten Behälter vorgesehen ist.

10. Kit nach Anspruch 7, ferner umfassend ein zu mischendes drittes Material.

11. Kit nach Anspruch 7, ferner umfassend eine Ausgabespitze (145), die an dem ersten Ende der Mischkammer fixiert werden kann.

12. Kit nach Anspruch 7, wobei der Mischkopf einen äußeren Ring (25) enthält, der eine Außenwand aufweist, und der äußere Ring mindestens einen sich radial erstreckenden Vorsprung enthält, der sich von der Außenwand des äußeren Rings erstreckt.

## Revendications

1. Appareil de mélange comprenant :
i. une chambre de mélange (60) comportant une première extrémité et une seconde extrémité, où ladite première extrémité est ouverte ;
ii. un dispositif de mélange fixé à ladite première extrémité, ledit dispositif de mélange comprenant un piston (1), une tige (7), une tête malaxeuse (8) et un dispositif (3) pour commander la rotation de la tête malaxeuse,
dans lequel ladite tête malaxeuse est introduite au moins partiellement dans la chambre de mélange ;
dans lequel ledit dispositif pour commander la rotation de la tête malaxeuse est configuré pour faire tourner la tête malaxeuse quand le piston se déplace dans une première direction axiale (10), et pour empêcher la tête malaxeuse de tourner quand le piston se déplace dans une seconde direction axiale (9) ;
dans lequel ladite chambre de mélange est le corps d'une première seringue et ladite première seringue comprend un piston (75) à sa seconde extrémité,
**caractérisé en ce que** :
la tête malaxeuse (8) comporte une pluralité de lames (15) obliques, et
ladite tête malaxeuse comprend une pluralité de protubérances (50) s'étendant axialement sur au moins une surface d'au moins une lame oblique sur ladite tête malaxeuse.

2. Appareil selon la revendication 1, comprenant en outre une pointe distributrice (145) pouvant être fixée à ladite première extrémité de ladite chambre de mélange.

3. Appareil selon la revendication 1, dans lequel ladite tête malaxeuse comprend un anneau extérieur (25) comportant une paroi extérieure et ledit anneau extérieur comprend au moins une protubérance s'étendant radialement depuis la paroi extérieure de l'anneau extérieur.

4. Procédé de mélange de deux composants comprenant les opérations consistant à :
i. avoir un premier composant et un second composant dans une chambre de mélange (60) comportant une première extrémité et une seconde extrémité, où ladite première extrémité est ouverte ;
ii. fixer un dispositif de mélange à ladite première extrémité, ledit dispositif de mélange comprenant un piston (1), une tige (7), une tête malaxeuse (8) et un dispositif (3) pour commander la rotation de la tête malaxeuse,
dans lequel ladite tête malaxeuse est introduite au moins partiellement dans la chambre de mélange ; et
iii. déplacer ledit piston dans une première direction axiale (10) et dans une seconde direction axiale (9) au moins une fois dans chacune,
dans lequel ledit dispositif pour commander la rotation de la tête malaxeuse fait tourner la tête malaxeuse quand le piston se déplace dans une première direction axiale, et empêche la tête malaxeuse de tourner quand le piston se déplace dans une seconde direction axiale ;
dans lequel ladite chambre de mélange est le corps d'une première seringue et ladite première seringue comprend un piston (75) à sa seconde extrémité,
**caractérisé en ce que** :
ladite tête malaxeuse (8) comporte une pluralité de lames (15) obliques, et
ladite tête malaxeuse comprend une pluralité de protubérances (50) s'étendant axialement sur au moins une surface d'au moins une lame oblique sur ladite tête malaxeuse.

5. Procédé selon la revendication 4, comprenant en outre une pointe distributrice (145) pouvant être fixée à ladite première extrémité de ladite chambre de mélange.

6. Procédé selon la revendication 4, dans lequel ladite tête malaxeuse comprend un anneau extérieur (25) comportant une paroi extérieure et ledit anneau extérieur comprend au moins une protubérance s'étendant radialement depuis la paroi extérieure de l'anneau extérieur.

7. Kit comprenant :
i. une chambre de mélange (60) comportant une première extrémité et une seconde extrémité, où ladite première extrémité est ouverte, dans lequel ladite chambre de mélange est le corps d'une première seringue et ladite première seringue comprend un piston (75) à sa seconde extrémité ;
ii. un dispositif de mélange pouvant être fixé à ladite première extrémité, ledit dispositif de mélange comprenant un piston (1), une tige (7), une tête malaxeuse (8) et un dispositif (3) pour commander la rotation de la tête malaxeuse,
dans lequel ladite tête malaxeuse est introduite au moins partiellement dans la chambre de mélange ;
dans lequel ledit dispositif pour commander la rotation de la tête malaxeuse est configuré pour faire tourner la tête malaxeuse quand le piston se déplace dans une première direction axiale (10), et pour empêcher la tête malaxeuse de tourner quand le piston se déplace dans une seconde direction axiale (9) ;
iii. une première matière à mélanger ; et
iv. une deuxième matière à mélanger,
**caractérisé en ce que** :
ladite tête malaxeuse (8) comporte une pluralité de lames (15) obliques, et
ladite tête malaxeuse (8) comprend une pluralité de protubérances (50) s'étendant axialement sur au moins une lame oblique sur ladite tête malaxeuse.

8. Kit selon la revendication 7, dans lequel ladite première matière est contenue dans ladite chambre de mélange.

9. Kit selon la revendication 7, dans lequel la deuxième matière à mélanger est disposée dans un récipient séparé.

10. Kit selon la revendication 7, comprenant en outre une troisième matière à mélanger.

11. Kit selon la revendication 7, comprenant en outre une pointe distributrice (145) pouvant être fixée à ladite première extrémité de ladite chambre de mélange.

12. Kit selon la revendication 7, dans lequel ladite tête malaxeuse comprend un anneau extérieur (25) comportant une paroi extérieure et ledit anneau extérieur comprend au moins une protubérance s'étendant radialement s'étendant depuis la paroi extérieure de l'anneau extérieur.
